# EUROPÄISCHE PATENTSCHRIFT

(11) **EP 0 587 984 B1**
(45) Veröffentlichungstag und Bekanntmachung des Hinweises auf die Patenterteilung: **03.09.1997**
(21) Anmeldenummer: 93107249.0
(22) Anmeldetag: 05.05.1993
(51) Int. Cl.: A61M 25/01

(54) **Dispenser mit einem Führungsdraht**
Dispenser with guidewire
Distributeur avec fil de guidage

(30) Priorität: 18.09.1992 DE 9212575 U
(43) Veröffentlichungstag der Anmeldung: 23.03.1994
(73) Patentinhaber: B. Braun Melsungen AG, 34209 Melsungen (DE)
(72) Erfinder: Pingel, Jörg, Dipl.-Oec., W-3501 Edermünde-Besse (DE); Schmidt, Klaus, W-3501 Ahnatal (DE); Wiegel, Heinz, Dipl.-Ing., W-6445 Alheim-Heinebach (DE); Göbel, Udo, Dipl.-Ing., W-3508 Melsungen-Kirchhof (DE)
(74) Vertreter: Selting, Günther, Dipl.-Ing.

(56) Entgegenhaltungen:
- US-A- 4 616 648
- US-A- 4 758 218
- US-A- 4 860 757
- US-A- 4 995 872
- US-A- 5 125 906

## Beschreibung

Die Erfindung bezieht sich auf einen Dispenser gemäß dem Oberbegriff von Anspruch 1.

Zur Einführung eines ein- oder mehrlumigen Katheters in ein Blutgefäß wird mit einem Führungsdraht gearbeitet, der durch eine Punktionsstelle bis zum Zielort gebracht wird und über den anschließend der Katheter vorgeschoben wird. Es handelt sich hierbei um die sogenannte Seldinger-Technik, die mit einem Seldingerdraht praktiziert wird, der sich durch eine hochflexible, J-förmig gebogene Spitze auszeichnet. Da Führungsdrähte eine beträchtliche Länge haben können (0,4 bis >1m) und bis auf die hochflexible Spitze eine gewisse federelastische Steifigkeit haben, wird der Anwender von dem frei aus dem Patientenkörper vorstehenden Führungsdrahtende behindert und es ist wichtig, dieses Ende in Schlaufenform zu halten. Zu diesem Zweck ist der Führungsdraht in einem spiralförmig verlegten und in dieser Form gehaltenen steifen Dispenserschlauch untergebracht, aus dem er durch ein Einführungsbesteck in den Patientenkörper abgegeben wird. Der Dispenserschlauch besteht aus festem Kunststoff, insbesondere Polypropylen, und sein Innendurchmesser ist größer als der Außendurchmesser des Führungsdrahtes, so daß dieser sehr leichtgängig im Schlauchlumen gleitet. Zur Streckung der J-förmigen Spitze des Führungsdrahtes dient dabei ein Einführstück am Übergang zwischen Dispenserschlauchende und Einlaß des Katheterbestecks, z.B. einem Kanülenansatz. Das Einführstück ist mit einer Röhre versehen, deren Innendurchmesser nur geringfügig größer ist als der Durchmesser des Führungsdrahtes, so daß die J-förmige Spitze gerade gestreckt wird.

US-A-4 758 218 beschreibt ein Gerät zum Legen eines Katheters mit Hilfe eines Führungsdrahtes. Das Gerät besteht aus einer Spritze, wobei der Spritzenkolben eine zentrale Ausnehmung aufweist, durch die der Führungsdraht verläuft. Am aus dem Spritzenkörper herausragenden Ende des Spritzenkolbens ist ein Umhüllungsschlauch, der den Führungsdraht umgibt, befestigt. Der Führungsdraht endet kurz vor der Nadel der Spritze. Das andere Ende befindet sich in dem Umhüllungsschlauch. Um den Führungsdraht in eine Vene des Patienten einzuführen, wird zunächst die Nadel in die Vene eingestochen. Zur Kontrolle, ob die Vene getroffen wurde, wird der Pumpenkolben zurückgezogen, so daß bei ordnungsgemäßem Ansetzen der Spritze das Blut sich in den Spritzenkörper ergießt. Nun wird der Umhüllungsschlauch entfernt und der Führungsdraht durch die Nadel hindurch in die Vene eingeführt. Ist der Führungsdraht tief genug in der Vene verankert, kann das komplette Gerät entfernt werden, während der Führungsdraht in der Vene verbleibt. Abschließend wird der Katheter über den Führungsdraht in die Vene gebracht.

Ein aus der Praxis bekanntes Einführstück ist als kreiszylindrische Röhre ausgebildet, die an einem Ende einen Konnektor-Außenkonus aufweist, der in das stumpf abgeschnittene offene Ende des Dispenserschlauches passend eingesteckt wird. Der spiralförmig verlegte Dispenserschlauch wird mit Hilfe separater längsgeschlitzter Clips in Stellung gehalten, die als Doppelspangen gestaltet sind und jeweils nebeneinanderliegende Schlauchstränge zusammenklammern. Um ein Herausrutschen des Führungsdrahtes aus dem rückwärtigen Ende des Dispenserschlauches zu verhindern, wird dieses Ende verschlossen. Hierzu wird es entweder verklebt oder verschweißt oder es wird mit einem separaten Stopfen verschlossen, der in das rückwärtige Ende des Dispenserschlauches festsitzend eingesteckt wird (DE-GM 81 12 065). In verpacktem Zustand steht die J-förmige Spitze des Führungsdrahtes über die vordere Mündung des Kanals der Röhre vor, damit die Krümmung ihren ursprünglichen Radius behält und nicht im Laufe der Lagerzeit durch permanente Streckung in der Röhre ermüdet. Um die J-Spitze des Führungsdrahtes in eine Kanüle einführen zu können, muß der Anwender mit einer Hand den Dispenserschlauch halten und mit der zweiten Hand das Einführstück fassen und aus dem Schlauchende ziehen. Dann muß der Anwender mit der Hand, die den Dispenserschlauch hält, den Führungsdraht zusätzlich festhalten und mit der anderen Hand das Einführstück über die J-Spitze ziehen, so daß das J-förmige Ende in dem Einführstück gestreckt wird. Hiernach führt der Anwender einen Luerkegel des Einführstückes, mit vollständig zurückgezogenem Führungsdraht, in den Ansatz der bereits punktierten Kanüle ein. Während er mit der einen Hand Einführstück und Kanülenende hält, kann er mit der anderen Hand den Führungsdraht in die Vene vorschieben. Diese Handhabung ist umständlich. Der bekannte Dispenser ist durch den Zusammenbau aus Einzelteilen teuer in der Herstellung und Montage. Außerdem ist besonders nachteilig, daß der Führungsdraht während des Transportes aus dem Einführstück frei herausrutschen kann und in der Verpackung abknickt. Der Einsatz des Führungsdrahtes wird dadurch erschwert oder sogar unmöglich gemacht.

Die letzterwähnten Nachteile hat auch ein aus US-5 125 906 bekannter Dispenser, der sich im übrigen durch Einhandbedienbarkeit auszeichnet. Diese ist darauf zurückzuführen, daß ein Axialabschnitt des Einführstückes zwischen einem hülsenförmigen Anschlußteil mit geschlossener Wand und der Röhre von einer Vorschubfläche überbrückt und nach außen offen ist, so daß mit einem Finger oder dem Daumen der das Einführstück haltenden Hand der den Axialabschnitt durchquerende Führungsdraht vorschiebbar ist. Der Röhrenkanal und der Führungsdraht-Durchlaß sind koaxial angeordnet, wodurch eine ungebremste leichtgängige Verschiebbarkeit des Führungsdrahtes gegeben ist, die während des Transportes des Dispensers ein Herausrutschen des Führungsdrahtes aus dem Dispenser und sein Abknicken in der Verpackung zur Folge haben kann.

Der Erfindung liegt die Aufgabe zugrunde, einen Dispenser mit einem Führungsdraht so zu verbessern, daß der Führungsdraht bei Lagerung und Transport sicher in dem Dispenserschlauch verbleibt, so daß er nicht abknicken kann.

Diese Aufgabe wird erfindungsgemäß dadurch gelöst, daß die Längsachsen des Führungsdraht-Durchlasses des Anschlußteiles und des mit axialem Abstand zu diesem angeordneten Kanals zur Erzielung eines gekröpft abgelenkten Bereiches des Führungdrahtes gegeneinander versetzt sind.

Die Versetzung der Ebenen von Führungsdraht-Durchlaß und Röhrenkanal hat zur Folge, daß der Führungsdraht im Bereich des Einführstückes aus seinem geraden Verlauf gekröpft abgelenkt ist, wodurch sich ein Selbsthemmeffekt für den in dem schleifenförmig verlegten Dispenserschlauch des Dispensers untergebrachten Führungsdraht ergibt, der sein Herausrutschen oder Verrutschen bei Lagerung und Transport verhindert. Die aus der Röhre vorstehende J-Spitze behält ihre Position und Form in der Verpackung bei. Es wird zuverlässig ausgeschlossen, daß der Anwender bei Öffnung einer Packung einen abgeknickten Führungsdraht antrifft und der Einsatz des Führungsdrahtes kann präzise und für den Patienten sicher erfolgen.

In vorteilhafter Ausgestaltung der Erfindung ist vorgesehen, daß in einem Axialabschnitt des Einführstückes zwischen dem Anschlußteil und der Röhre eine von außen frei zugängliche schräge Vorschubfläche für den Führungsdraht angeordnet ist, die von dem Anschlußteil zu der Röhre nach vorne schräg geneigt verläuft und konvex gewölbt ist.

Vorzugsweise befindet sich der Anschlußteil oben und die Röhre unten, so daß die Vorschubfläche von hinten nach vorne abwärts geneigt verläuft und der Führungsdraht entsprechend gebogen ist. Die versetzten Ebenen können parallel oder in einem stumpfen Winkel zueinander verlaufen. Entsprechend sind die Längsachsen der Führungsdrahtdurchlässe orientiert.

Der in dem Axialabschnitt zwischen Anschlußteil und Röhre frei verlaufende Führungsdraht wird mit dem Daumen oder Finger einer Hand über die schräge Vorschubfläche geschoben, wobei der im Ruhezustand des Dispensers zur Verhinderung des Verrutschens ausreichende Bremseffekt problemlos überwunden wird, so daß der Führungsdraht sich zur Streckung der J-Spitze in die Röhre hinein und zur Einführung in eine Kanüle leichtgängig aus der Röhre und dem Dispenserschlauch herausschieben läßt. Der Dispenser ist für Rechts- und Linkshänder ausgelegt und erlaubt eine rechts- und linksseitige Gefäßpunktion.

Vorteilhafterweise ist der das hintere Schlauchende festhaltende Clip an dem Einführstück als Tasche ausgebildet, die am hinteren stirnseitigen Ende verschlossen ist. Der Verschluß bildet einen Anschlag für den Dispenserschlauch und den in Anwendungsposition befindlichen Führungsdraht. Zum endseitigen Verschluß des Clips kann dieser mit einer Stirnwand versehen sein. Wenn das vordere Ende des Dispenserschlauches mit dem Anschlußteil des Einführstückes zusammengesteckt ist und das hintere Ende des Dispenserschlauches in der Tasche des endseitig verschlossenen Clips gehalten ist, wird der Führungsdraht im Anwendungszustand der Anordnung mit in die Röhre eingezogener, gestreckter Führungsdrahtspitze an einem Herausrutschen nach hinten von der Stirnwand gehindert, so daß nicht versehentlich die Spitze des Führungsdrahtes durch zu weites Zurückziehen aus dem hinteren Ende des Kanals der Röhre herausgleiten kann. Der Verschluß des Clips erleichtert damit die Anwendung des Dispensers, da die Länge des Führungsdrahtes dem Wegstück zwischen Stirnwand des Clips und Auslaßmündung der Röhre angepaßt ist. Zu Beginn der Anwendung des Dispensers wird zur Streckung der J-Spitze des Führungsdrahtes der Führungsdraht im Dispenserschlauch soweit zurückgeschoben, bis das hintere Führungsdrahtende gegen die Stirnwand des Clips stößt und das vordere Führungsdrahtende in der Röhrenmündung verschwunden ist. Sodann wird die Mündung der Röhre mit einem Ansatz einer bereits verlegten Kanüle zusammengebracht und es wird mit einer Hand der Führungsdraht über die schräge Vorschubfläche vorgeschoben, so daß er durch die mit der anderen Hand festgehaltene Kanüle in die Vene vordringt.

Die Montage des Dispensers ist einfach. Dieser Vorteil wird dadurch vergrößert, daß auch der Anschlußteil für das vordere Ende des Schlauches als längsgeschlitzter Clip gestaltet ist und daß die beiden dem Durchmesser des Schlauches angepaßten Längsschlitze in zur Ebene der Schlauchschleife senkrechten Ebenen liegen. Diese seitliche Anordnung der Längsschlitze der Clips erlaubt eine Verbindung von Dispenserschlauch und Einführstück durch einfaches Hineindrücken der Schlauchenden in die Clips zur festen Verbindung der Teile miteinander. Anschließend wird der Führungsdraht eingefädelt und der Dispenser ist fertig montiert.

Zur Erzielung einer schnellen und haltbaren Verbindung zwischen der Mündung der Röhre und einem Einführbesteck, z.B. dem Ansatz einer Kanüle, ist an der vorderen Mündung der Röhre ein Konnektor-Außenkonus (Luer-Kegel) ausgebildet. Hierdurch wird ein exakter Sitz des Einführstückes im Kanülenansatz und ein störungsfreier Übergang der Spitze des Führungsdrahtes aus der Röhre in die Kanüle erreicht.

In der Zeichnung sind zwei Ausführungsbeispiele der Erfindung schematisch dargestellt. Es zeigt:
Fig. 1 eine Draufsicht auf eine erste Ausführungsform eines Dispensers mit eingelegtem Führungsdraht im verpackten Originalzustand,
Fig. 2 einen Schnitt durch das Einführstück nach Fig. 1 mit Endabschnitten des Dispenserschlauches,
Fig. 3 eine Draufsicht auf die Anordnung nach Figur 2 in Richtung des Pfeiles III gesehen,
Fign. 4, 5 und 6 Querschnitte entlang der Linien IV-IV, V-V, VI-VI in Figur 2,
Fig. 7 einen Schnitt durch das Einführstück im Zustand unmittelbar vor der Anwendung,
Fig. 8 eine Draufsicht auf eine zweite Ausführungsform eines Dispensers mit eingelegtem Führungsdraht im verpackten Originalzustand,
Fig. 8A einen Abschluß- und Halteclip für den Dispenserschlauch in perspektivischer Ansicht,
Fig. 9 eine Ansicht des Einführstückes ohne Führungsdraht, gesehen in Richtung des Pfeiles IX in Figur 8 und
Fig. 10 eine Draufsicht auf das Einführstück ohne Führungsdraht, gesehen in Richtung des Pfeiles X in Figur 8.

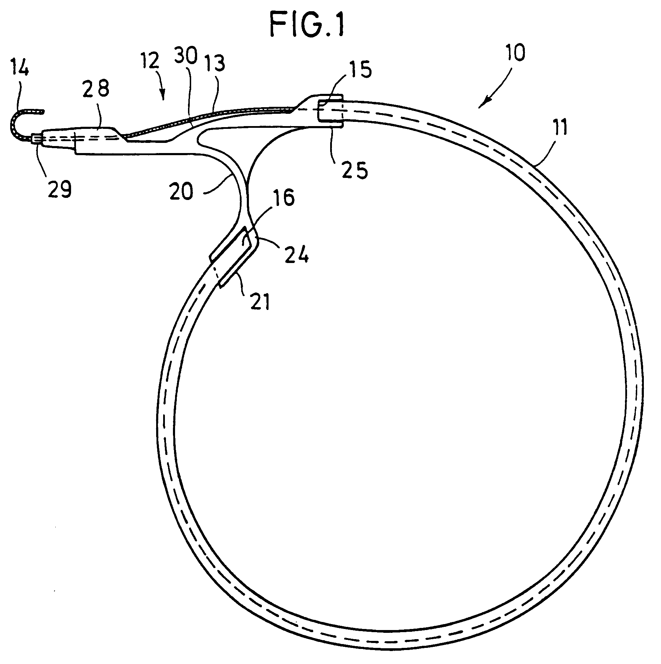

Gemäß Figuren 1 bis 7 besteht ein Dispenser 10 im wesentlichen aus einem Dispenserschlauch 11 mit einheitlichem Durchmesser und einem Einführstück 12, die miteinander verbunden sind, sowie aus einem Führungsdraht 13 mit J-förmig gebogener flexibler Spitze 14 (Fig. 1).

Der Dispenserschlauch 11 mit kreisförmigem Außen- und Innendurchmesser aus Kunststoff, vorzugsweise Polypropylen, läßt sich ohne Veränderung seines Innenquerschnittes zu einer Schleife, insbesondere einer angenäherten Spirale, verlegen. Das eine, im vorliegenden Falle vordere, Ende 15 des Dispenserschlauches 11 ist stumpf abgeschnitten, während das andere, im vorliegenden Falle hintere, Ende 16 abgeschrägt ist.

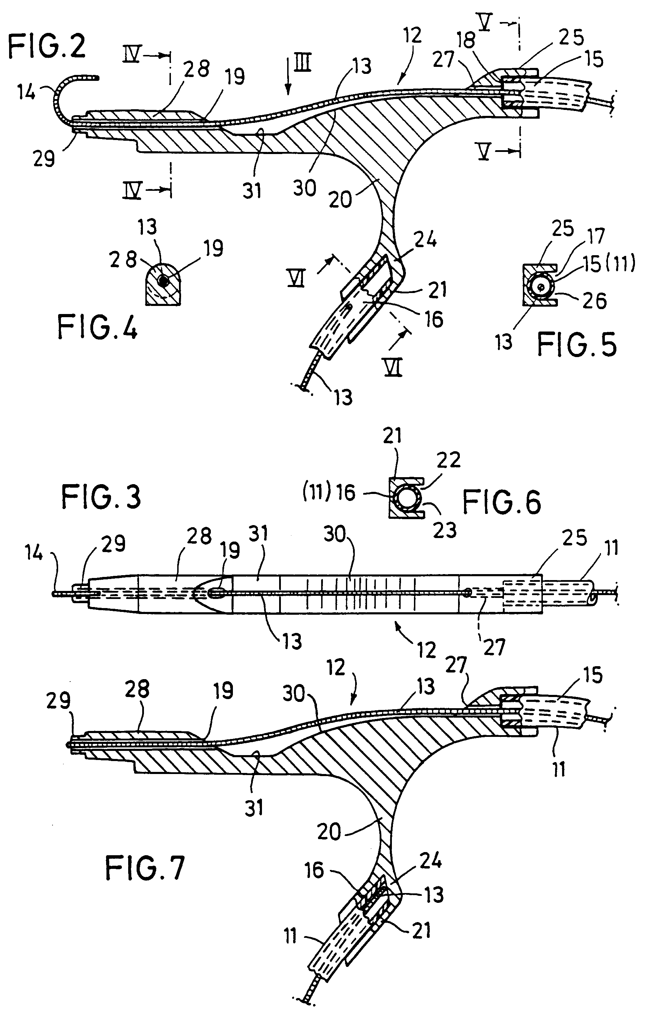

Das Einführstück 12, das als Spritzgußteil aus hartem Kunststoff gefertigt ist, hat die Form einer abgeflachten Pistole. Von einem langgestreckten Korpus geht in gleicher Ebene nach einer Seite ein konkav gewölbter Griff 20 aus, der am unteren Ende angeformt einen schräg gerichteten Clip 21 trägt, welcher im Querschnitt U-förmig und an einer Stirnseite offen ist. Der Clip 21 hat einen der Form des Dispenserschlauchendes 16 mit leichtem Untermaß angepaßten Hohlraum 22 (Fig. 6), der an einer Längsseite mittels eines Längsschlitzes 23 geöffnet ist. Der Längsschlitz 23 gestattet ein seitliches Hineindrücken des Endes 16 des Dispenserschlauches 11 in den Hohlraum 22 des Clips 21. Der Längsschlitz 23 befindet sich in der zur Ebene des Einführstück-Korpus senkrechten Ebene, d.h., der Hohlraum 22 öffnet sich gegen den Betrachter der Zeichnung. Das in Vorschubrichtung des Endes 16 des Dispenserschlauches 11 vordere Ende des Hohlraumes 22 des Clips 21 ist durch eine Stirnwand 24 verschlossen, deren Innenfläche so abgeschrägt ist, daß die Schräge des hinteren Endes 16 des Dispenserschlauches 11 bündig anliegt, wenn der Dispenserschlauch 11 sich im montierten Zustand befindet.

Ein dem Clip 21 ähnlicher Clip 25 ist am hinteren Ende des langgestreckten Korpus des Einführstückes 12 ausgebildet. Dieser Clip 25 bildet einen Anschlußteil für das vordere Ende 15 des Dispenserschlauches 11. Auch er besitzt einen dem Dispenserschlauch 11 mit leichtem Untermaß angepaßten U-förmigen Hohlraum 17, der an einer Stirnseite offen und an einer Längsseite, und zwar der gleichen Seite wie bei Clip 21, durch einen Längsschlitz 26 geöffnet ist, damit das Ende 15 des Dispenserschlauches 11 sich klemmend in den Clip 25 seitlich hineindrücken läßt. Die Montage des Dispenserschlauches 11 wird durch die beiden auf der gleichen Seite längsgeschlitzten Clips 21 und 25 erleichtert.

In einer vorderen Stirnwand 18 des Clips 25 befindet sich eine zentrale gerade Bohrung 27, die ein leichtgängiges Hindurchgleiten des Führungsdrahtes 13 erlaubt. In axialem Abstand zu dem vorderen Ende der Mündung des Führungsdraht-Durchlasses 27 ist eine Mündung eines geraden Kanals 19 einer Röhre 28 in dem Einführstück 12 vorgesehen. Die Röhre 28 verläuft gerade und ihre Längsachse ist in einer zu der Längsachse des Führungsdraht-Durchlasses 27 parallel versetzten Ebene angeordnet. Die Versetzung ist so vorgesehen, daß bei Erfassen des Einführstückes 12 durch Einlegen des Fingers der einen Hand in die Mulde des Griffes 20 die Röhre 28 tiefer liegt als der Führungsdraht-Durchlaß 27. An der vorderen Mündung des Kanals 19 der Röhre 28 ist ein Konnektor-Außenkonus 29 ausgebildet, der ein sicheres Einstecken des Einführstückes 12 in den Ansatz einer Kanüle erlaubt und seinen exakten Sitz gewährleistet.

Das Abstandsstück zwischen der Röhre 28 und dem Führungsdraht-Durchlaß 27 ist nach oben und seitwärts offen und wird unten von einer schrägen Vorschubfläche 30 eingenommen, die sich konvex gewölbt von der Ausmündung des Führungsdraht-Durchlasses 27 nach unten zu der Einmündung des Kanals 19 der Röhre 28 erstreckt, wobei die konvexe Wölbung unten an einem geraden waagerechten Endabschnitt 31 ausläuft, der etwas unterhalb der Scheitellinie des Kanals 19 der Röhre 28 liegt. Die vordere Fläche der Stirnwand 18 des Clips 25 und die hintere Fläche der Röhre 28 sind abgeschrägt und divergieren nach oben.

Der Führungsdraht 13 mit flexibler J-förmig gebogener Spitze 14 wird in den montierten Dispenser 10 so eingeschoben, daß die flexible J-Spitze 14 aus dem Konnektor-Außenkonus 29 des Einführstückes 12 herausragt und über diesen vorsteht. Das gerade hintere Ende des Führungsdrahtes 13 ist dadurch in einer korrespondierenden Länge vom Anschlag 24 des Clips 21 entfernt und berührt diesen nicht. In diesem Zustand wird der Dispenser 10 mit Führungsdraht 13 verpackt (Fign. 1,2,3). Durch den Höhenversatz zwischen den Längsachsen des Führungsdraht-Durchlasses 27 und des Kanals 19 der Röhre 28 ergibt sich ein Selbsthemmeffekt für den Führungsdraht 13, so daß dieser bei Lagerung und Transport nicht aus dem Dispenser 10 weiter nach vorne herausrutschen und abknicken kann.

Bei der Anwendung des der Verpackung entnommenen Dispensers 10 gemäß Figuren 1, 2, 3 drückt der Anwender den Führungsdraht 13 mit dem Daumen der einen Hand gegen die Vorschubfläche 30 des Einführstückes 12 und schiebt ihn im Dispenserschlauch 11 bis zum Anschlag gegen die Stirnwand 24 in die Position gemäß Figur 7. Bei dem Abstand zwischen der Stirnwand 24 und der vorderen Mündung des Konnektor-Außenkonus 29 angepaßter Länge des Führungsdrahtes 13 ist die flexible J-Spitze 14 sodann in der Röhre 28 zurückgezogen und wird in dieser gerade gerichtet gehalten. Anschließend wird der Luer-Konus 29 in den Ansatz einer in Punktionsposition befindlichen Kanüle eingeführt, so daß er mit dieser sicher verbunden ist. Nun wird mit dem Daumen der das Einführstück 12 haltenden Hand der Führungsdraht 13 über die Vorschubfläche 30 in die Vene vorgeschoben, während die andere Hand die Kanüle festhält.

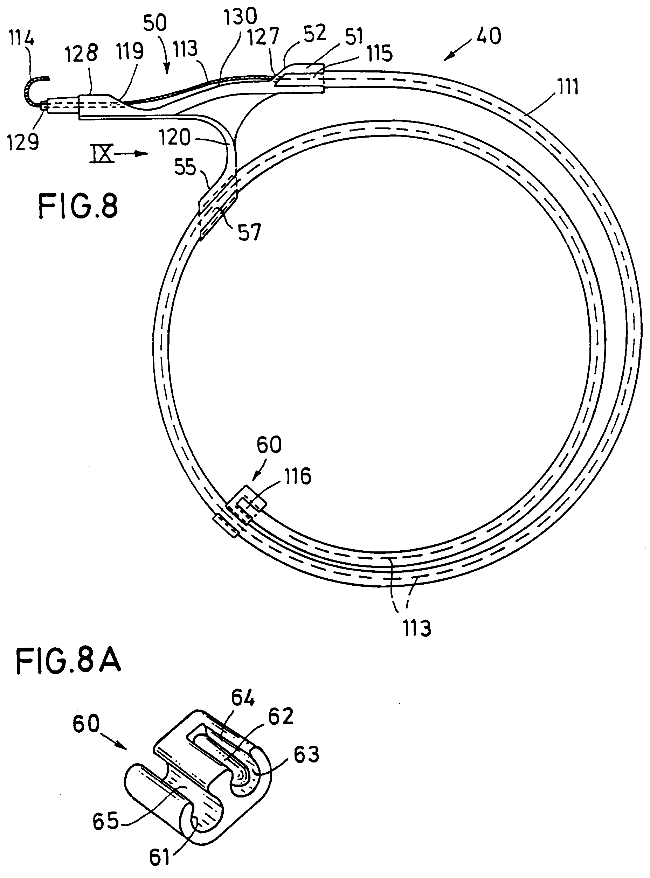

Das Beispiel der Figuren 8 bis 10 zeigt eine zweite Ausführungsform der Erfindung, bei der ein Führungsdraht 113 mit flexibler J-förmiger Spitze 114 und ein Dispenserschlauch 111 so lang sind, daß eine Spiralverlegung in etwa doppelter Kreisführung möglich und nötig ist. Bauteile, die dem Dispenser 10 des Beispiels der Figuren 1 bis 7 entsprechen, sind mit gleichen Bezugszeichen, jedoch unter Zusatz von "100" gekennzeichnet.

Ein Dispenser 40 dieses Beispiels besteht aus einem langen Dispenserschlauch 111 mit einheitlichem Durchmesser, einem mit diesem verbundenen Einführstück 50 und einem Führungsdraht 113 mit J-förmig gebogener flexibler Spitze 114 (Figur 8).

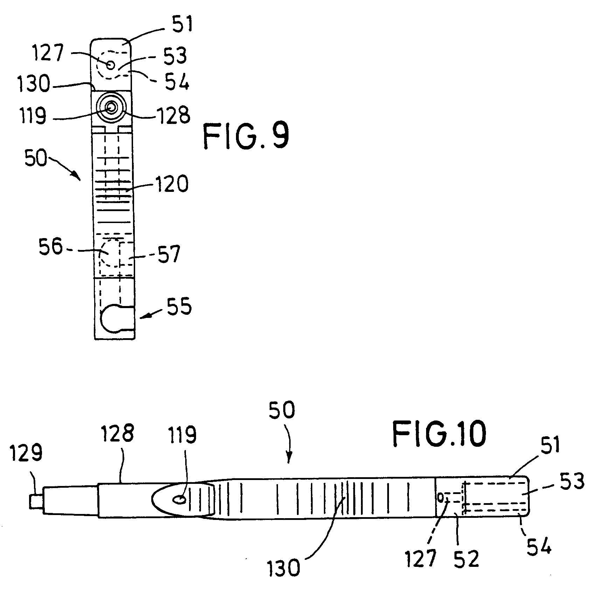

Der Dispenserschlauch 111 ist vorzugsweise aus Polypropylen hergestellt. Sein vorderes Ende 115 ist schräg abgeschnitten und sein hinteres Ende 116 endet stumpf. Das Einführstück 50 ist ähnlich dem Einführstück 12 ausgebildet und aus hartem Kunststoff gefertigt. Ein langgestreckter Korpus weist an einem Ende eine gerade Röhre 128 auf, die von einem geraden Kanal 119 durchsetzt ist, der am vorderen Ende in einem Konnektor-Außenkonus 129 ausmündet und am hinteren Ende gegen eine freie Profilfläche öffnet, die eine Vorschubfläche 130 für den Führungsdraht 113 bildet. Die Vorschubfläche 130 verläuft von der hinteren Mündung des Kanals 119 konvex geschwungen nach oben zu einem geraden Führungsdraht-Durchlaß 127, der in einer vorderen Stirnwand 52 eines längsgeschlitzten Clips 51 ausgebildet ist. Die Stirnwand 52 ist zumindest innen schräg gerichtet, so daß das schräg abgeschnittene Ende 115 des Dispenserschlauches 111 passend gegen die Innenfläche zur Anlage kommt. Der Kanal 119 der Röhre 128 und der Führungsdraht-Durchlaß 127 verlaufen in zueinander parallel versetzten Ebenen, wobei die Röhre 128 sich in Anwendungshaltung des Dispensers 40 unten befindet. Der Clip 51 hat einen Hohlraum 53 mit dem Umfang des Dispenserschlauches 111 mit Untermaß angepaßtem Teilkreisquerschnitt, in den ein parallelrandiger Längsschlitz 54 übergeht (Figur 9). Das in den Hohlraum 53 durch den Längsschlitz 54 hineingedrückte Ende 115 des Dispenserschlauches 111 ist in dem Clip 51 klemmend festgehalten.

Von dem langgestreckten Korpus des Einführstückes 50 steht nach einer Seite, und zwar im Anwendungszustand nach unten, ein konkav gewölbter Griff 120 ab, an dessen unteres Ende ein zweiter längsgeschlitzter Clip 55 angeformt ist. Der Hohlraum 56 dieses Clips 55 ist an beiden Stirnseiten offen, so daß eine federnde Spannklaue zur Aufnahme eines Mittelbereiches des langen, spiralförmig verlegten Dispenserschlauches 111 entsteht. Der Axialdurchgang des Clips 55 ist unter einem Winkel von etwa 50°, vorzugsweise 48°, zur Horizontalen schräg gerichtet, so daß er dem Verlauf der Schleife des Dispenserschlauches 111 folgt und dieser bei der Weiterführung seines hinteren Abschnittes im Innern der Spirale nicht abknickt. Das Querschnittsprofil des Hohlraumes 56 des Clips 55 entspricht demjenigen des Hohlraumes 53 des Clips 51. Wie Figur 9 zeigt, ist der Hohlraum 56 in Anpassung an den Umfang des Schlauches 111 teilkreisförmig. Durch einen parallelrandigen Längsschlitz 57 läßt sich der Dispenserschlauch 111 von der Seite her in den Clip 55 hineindrücken. Die beiden Längsschlitze 54 und 57 der Clips 51 und 55 sind in Draufsicht auf das Einführstück 50 nach der gleichen Seite gerichtet (Fign. 8,9).

Das hintere Ende 116 des Dispenserschlauches 111 der innenliegenden Spiralenschleife wird an dem benachbarten äußeren Schlauchteil der Spiralenschleife mit Hilfe eines Clips 60 festgemacht, der parallel neben einem an beiden Enden offenen Klauenkörper 61 einen stopfenförmigen Verschlußkörper 62 für das Schlauchende aufweist. Der stopfenförmige, kreiszylindrische Verschlußkörper 62 ist mit radialem Abstand von einer zylindrischen Teilkreisfläche 63 umgeben, die an einem Längsschlitz 64 offen ist. Der Längsschlitz 64 befindet sich an der gleichen Seite wie ein Längsschlitz 65 des Klauenkörpers 61. Der Verschlußkörper 62 paßt klemmend in das Lumen des Dispenserschlauches 111 und verschließt sein Ende 116, wobei die Teilkreisfläche für zusätzlichen Halt sorgt.

Die Länge des Führungsdrahtes 113 ist in bezug auf die Länge des Dispenserschlauches 111 mit angesetztem Einführstück 50 so bemessen, daß im verpackten Originalzustand (Figur 8) die flexible J-Spitze 114 über die Mündung der Röhre 128 vorsteht und das hintere Ende wie gezeichnet im Abstand zu dem Clip 60 liegt. Diese Position wird durch den Selbsthemmeffekt des Führungsdrahtes 113 an der Umlenkung zwischen Kanal 119 und Führungsdraht-Durchlaß 127 sowie Reibungsschluß zwischen Führungsdraht 113 und Vorschubfläche 130 gesichert. Bei der Anwendung des ausgepackten Dispensers 40 wird der Führungsdraht 113 zurückgeschoben, so daß sein Ende gegen den Verschlußkörper 62 anstößt und das Ende der flexiblen Spitze 114 in der Röhre 128 verschwunden ist, so wie es zu dem ersten Beispiel in Figur 7 gezeigt ist. Diese Handhabung ist einfach und sicher und nach Zusammenstecken des Konnektor-Außenkonus 129 in den Ansatz einer in Punktionsstellung befindlichen Kanüle wird die gestreckte Spitze 114 problemlos in die Kanüle und aus dieser in die Vene vorgeschoben, wobei die eine Hand die Kanüle festhält und die andere Hand den Dispenser 40 hält und den Führungsdraht 113 bewegt.

## Patentansprüche

1. Dispenser mit einem Führungsdraht (13;113), insbesondere einem Seldingerdraht mit J-förmig gebogener flexibler Spitze (14;114), bestehend aus einem den Führungsdraht (13;113) teilweise umhüllenden, schleifenförmig verlegten Schlauch (11;111), einem längsgeschlitzten Clip (21;60) zur Aufnahme des einen Endes (16;116) des Schlauches (11;111) und einem Einführstück (12;120), das ein Anschlußteil (25;51) mit Führungsdraht-Durchlaß (27;127) für das andere Ende (15;115) des Schlauches (11;111) und eine Röhre (28;128) mit geradem Kanal (19;119) zur Streckung der J-förmigen Spitze (14;114) des Führungsdrahtes (13;113) aufweist, wobei zwischen dem Führungsdraht-Durchlaß (27;127) und dem Kanal (19;119) ein Bereich vorhanden ist, in dem der Führungsdraht (13;113) frei zugänglich ist,
**dadurch gekennzeichnet,**
daß die Längsachsen des Führungsdraht-Durchlasses (27;127) und des mit axialem Abstand zu diesem angeordneten Kanals (19;119) zur Erzielung eines gekröpft abgelenkten Bereiches des Führungsdrahtes (13;113) gegeneinander versetzt sind.

2. Dispenser nach Anspruch 1, dadurch gekennzeichnet, daß die versetzten Längsachsen des Führungsdraht-Durchlasses (27) und des Kanals (19) parallel oder in einem stumpfen Winkel zueinander verlaufen.

3. Dispenser nach Anspruch 1 oder 2, dadurch gekennzeichnet, daß in einem Axialabschnitt des Einführstückes (12) zwischen dem Anschlußteil (25) und der Röhre (28) eine von außen frei zugängliche schräge Vorschubfläche (30) für den Führungsdraht (13) angeordnet ist, die von dem Anschlußteil (25) zu der Röhre (28) schräg zu dieser hin geneigt verläuft und konvex gewölbt ist.

4. Dispenser nach einem der Ansprüche 1 bis 3, **dadurch gekennzeichnet**, daß der das eine Schlauchende (16) festhaltende Clip (21) an dem Einführstück (12) als Tasche ausgebildet ist, die an der das eine Schlauchende (16) begrenzenden Stirnseite geschlossen ist.

5. Dispenser nach Anspruch 4, **dadurch gekennzeichnet**, daß der endseitige Verschluß des Clips (21) eine Stirnwand (24) ist.

6. Dispenser nach einem der Ansprüche 1 bis 3, **dadurch gekennzeichnet**, daß der längsgeschlitzte Clip (60) parallel neben einem an beiden Enden offenen Klauenkörper (61) einen Verschlußkörper (62) für das eine Ende des Schlauches (111) aufweist,
und daß ein an dem Einführstück (50) ausgebildeter längsgeschlitzter, endseitig offener Clip (55) den Schlauch (111) in einem Bereich zwischen seinen Enden klemmend umgreift.

7. Dispenser nach einem der Ansprüche 1 bis 6, **dadurch gekennzeichnet**, daß an der vorderen Mündung der Röhre (128) ein Konnektor-Außenkonus (Luer-Kegel 129) ausgebildet ist.

8. Dispenser nach einem der Ansprüche 1 bis 7, **dadurch gekennzeichnet**, daß auch der Anschlußteil (51) für das vordere Ende (115) des Schlauches (111) als längsgeschlitzter Clip gestaltet ist und daß die dem Durchmesser des Schlauches (111) angepaßten Längsschlitze (54,57) der Clips (51,55) in zur Ebene der Schlauchschleife senkrechten Ebenen liegen.

## Claims

1. A dispenser with a guide wire (13; 113), in particular with a Seldinger wire with a J-shaped bent flexible tip (14; 114), the dispenser consisting of a tube (11; 111) arranged in loop-shape and partially enclosing said guide wire (13; 113), a longitudinally slit clip (21; 60) for receiving the one end (16; 116) of said tube (11; 111), and an insertion member (12; 120) having a connector member (25; 51) with a guide wire passage (27; 127) for the other end (15; 115) of said tube (11; 111) and a pipe (28; 128) with a straight channel (19; 119) for straightening said J-shaped tip (14; 114) of said guide wire (13; 113), there being a region between said guide wire passage (27; 127) and said channel (19; 119), in which region said guide wire (13; 113) is freely accessible,
characterized in
that the longitudinal axes of said guide wire passage (27; 127) and said channel (19; 119) axially spaced therefrom are mutually offset for obtaining a cranked deflected portion of said guide wire (13; 113).

2. The dispenser of claim 1, characterized in that said offset longitudinal axes of said guide wire passage (27) and said channel (19) extend in parallel or under an obtuse angle with regard to one another.

3. The dispenser of claim 1 or 2, characterized in that in an axial section of said insertion member (12) between said connector member (25) and said pipe (28), an inclined feed surface (30) for the guide wire (13) is provided for free access from outside, the surface extending obliquely from said connector member (25) towards said pipe (28) and being convexly curved.

4. The dispenser of one of claims 1 to 3, characterized in that the clip (21) fixing said one tube end (16) is formed as a pocket at said insertion member (12), the pocket being closed at the end face defining said one hose end (16).

5. The dispenser of claim 4, characterized in that the end-side closure of said clip (21) is an end wall (24).

6. The dispenser of one of claims 1 to 3, characterized in that said longitudinally slit clip (60) has a closure member (62) for said one end of said tube (111) provided in parallel with a claw member (61) open at both ends, and that a longitudinally slit clip (55), formed at the insertion member (50) and open toward the end, clamps said tube (111) circumferentially in a region between the ends thereof.

7. The dispenser of one of claims 1 to 6, characterized in that a connector outer cone (Luer cone 129) is formed at the front end of said pipe (128).

8. The dispenser of one of claims 1 to 7, characterized in that also said connector member (51) for the front end (115) of said tube (111) is formed as a longitudinally slit clip, and that the longitudinal slits (54, 57) of said clips (51, 55), adapted to the diameter of said tube (111), are in planes perpendicular to the plane of the tube loop.

## Revendications

1. Distributeur muni d'un fil-guide (13; 113), notamment d'un fil de Seldinger présentant une pointe flexible (14; 114) recourbée en forme de J, constitué d'un tuyau souple (11; 111) disposé en forme de boucle et enveloppant partiellement le fil-guide (13; 113), d'un élément de pincement (21; 60) fendu longitudinalement et destiné à recevoir l'une des extrémités (16; 116) du tuyau souple (11; 111), et d'une pièce d'introduction (12; 120), qui présente une partie de raccordement (25; 51) avec un passage de fil-guide (27; 127) pour l'autre extrémité (15 ; 115) du tuyau souple (11; 111), et un tube (28; 128) comportant un canal rectiligne (19; 119) pour étendre la pointe en forme de J (14; 114) du fil-guide (13; 113), une zone dans laquelle le fil-guide (13; 113) est librement accessible, étant prévue entre le passage de fil-guide (27; 127) et le canal (19; 119),
caractérisé en ce que les axes longitudinaux du passage de fil-guide (27; 127) et du canal (19; 119) disposé à distance axiale de ce dernier, sont décalés l'un par rapport à l'autre pour l'obtention d'un tronçon à déviation contrecoudée du fil-guide (13; 113).

2. Distributeur selon la revendication 1, caractérisé en ce que les axes longitudinaux décalés du passage de fil-guide (27) et du canal (19) s'étendent parallèlement ou en formant un angle obtus l'un par rapport à l'autre.

3. Distributeur selon la revendication 1 ou 2, caractérisé en ce que dans un tronçon axial de la pièce d'introduction (12), entre la partie de raccordement (25) et le tube (28), est disposée une surface oblique d'avancement (30) pour le fil-guide (13), qui est librement accessible de l'extérieur, qui s'étend de la partie de raccordement (25) jusqu'au tube (28) en étant inclinée obliquement vers ce dernier, et qui présente une courbure convexe.

4. Distributeur selon l'une des revendications 1 à 3, caractérisé en ce que l'élément de pincement (21), qui maintient fermement l'une (16) des extrémités du tuyau souple, est réalisé sur la pièce d'introduction (12), sous la forme d'une cavité qui est fermée sur le côté frontal limitant ladite extrémité (16) du tuyau souple.

5. Distributeur selon la revendication 4, caractérisé en ce que la fermeture d'extrémité de l'élément de pincement (21) est une paroi frontale (24).

6. Distributeur selon l'une des revendications 1 à 3, caractérisé en ce que l'élément de pincement (60) fendu longitudinalement présente, à côté d'un corps à griffe (61) ouvert aux deux extrémités et parallèlement à celui-ci, un corps de fermeture (62) pour l'une des extrémités du tuyau souple (111), et en ce qu'un élément de pincement (55) fendu longitudinalement, ouvert aux extrémités et formé sur la pièce d'introduction (50), entoure en le serrant le tuyau souple (111) dans une zone entre ses extrémités.

7. Distributeur selon l'une des revendications 1 à 6, caractérisé en ce qu'à l'embouchure avant du tube (128) est formé un cône extérieur de connecteur (cône Luer 129).

8. Distributeur selon l'une des revendications 1 à 7, caractérisé en ce que la partie de raccordement (51) pour l'extrémité avant (115) du tuyau souple (111), présente également une configuration d'élément de pincement fendu longitudinalement, et en ce que les fentes longitudinales (54, 57) des éléments de pincement (51, 55), adaptées au diamètre du tuyau souple (111), se trouvent dans des plans perpendiculaires au plan de la boucle du tuyau souple.
